(19) Europäisches Patentamt
European Patent Office
Office européen des brevets

(11) Numéro de publication : **0 063 149**
**B1**

(12) # FASCICULE DE BREVET EUROPÉEN

(45) Date de publication du fascicule du brevet : **18.09.85**

(21) Numéro de dépôt : **81902972.9**

(22) Date de dépôt : **23.10.81**

(86) Numéro de dépôt international : **PCT/FR 81/00138**

(87) Numéro de publication internationale : **WO/8201467 (13.05.82 Gazette 82/12)**

(51) Int. Cl.⁴ : **A 61 K 9/00**, A 61 K 31/02, A 61 K 31/025, A 61 K 31/08, A 61 K 31/13, C 12 N 1/00

(54) **MICROEMULSIONS AQUEUSES DE FLUOROCARBURES INDEFINIMENT STABLES A UNE TEMPERATURE DONNEE, PROCEDE D'OBTENTION ET APPLICATIONS.**

**Jointe à la demande no. 81401690.3/0051526 (numéro de dépôt/numéro de publication de la demande européenne) par décision du 7.12.83.**

(30) Priorité : **24.10.80 FR 8119878**

(43) Date de publication de la demande : **27.10.82 Bulletin 82/43**

(45) Mention de la délivrance du brevet : **18.09.85 Bulletin 85/38**

(84) Etats contractants désignés : **AT BE CH DE FR GB IT LI LU NL SE**

(56) Documents cités :
**FR-A- 2 105 287**
**FR-A- 2 218 903**
**FR-A- 2 313 024**
**US-A- 3 778 381**
**US-A- 4 105 798**
**Chemical Abstracts, volume 83, no. 2, 14 juillet 1975 (Columbus Ohio, US), page 349, abrege 15661d**
**Le dossier contient des informations techniques présentées postérieurement au dépôt de la demande et ne figurant pas dans le présent fascicule.**

(73) Titulaire : **Etablissement Public dit: CENTRE NATIONAL DE LA RECHERCHE SCIENTIFIQUE (CNRS)**
**15, Quai Anatole France**
**F-75007 Paris (FR)**

(72) Inventeur : **MATHIS, Gérard**
**48 ter, avenue Anatole France**
**F-54000 Nancy (FR)**
Inventeur : **DELPUECH, Jean Jacques**
**16, rue du Plateau**
**F-54520 Laxou (FR)**

(74) Mandataire : **Phélip, Bruno et al**
**c/o Cabinet Harlé & Phélip 21, rue de la Rochefoucauld**
**F-75009 Paris (FR)**

**EP 0 063 149 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne des microémulsions aqueuses de fluorocarbures. Elle a également pour objet un procédé pour l'obtention de ces microémulsions et leur application à titre de transporteurs de gaz, notamment à titre de transporteur d'oxygène.

Il est connu que les fluorocarbures conviennent comme transporteurs d'oxygène dans les corps vivants [L. Clark et al. Triangle XIII n° 2 p. 85 1973] et que des émulsions de fluorocarbures ont déjà été utilisées comme substituts du sang [Proceedings of the IV$^{th}$ International Symposium on Perfluorochemicals blood substitutes. Ed. Excerpta Medica 1979].

Dans cette application des fluorocarbures, il est nécessaire de fabriquer des émulsions aqueuses stables de ces fluorocarbures de manière à véhiculer l'oxygène dissous dans le fluorocarbure et les sels et métabolites qui sont normalement présents dans le plasma. Il est connu que la toxicité des émulsions varie avec le pourcentage de particules égales ou supérieures à 0,4 µm. Par ailleurs, plus les particules sont fines, plus les émulsions sont efficaces. Il est important d'avoir des émulsions ayant des dimensions de particules relativement faibles, en tout état de cause inférieures à 0,2 µm, ou mieux encore inférieures à 0,1 µm, de manière à ce qu'elles puissent être compatibles avec la circulation dans l'organisme et être administrées par injection ou perfusion intra-veineuse.

On a déjà proposé des procédés pour l'obtention de telles émulsions. Par exemple, le brevet FR 71.31.983 (publié sous le n° 2.105.287) concerne un procédé pour l'obtention d'une émulsion d'un fluorocarbure pour injection capable de transporter de l'oxygène, qui consiste à émulsionner une solution aqueuse d'un fluorocarbure avec un agent tensio-actif, à centrifuger l'émulsion aqueuse résultante de façon à diviser les particules de fluorocarbure dans l'émulsion et à stériliser l'émulsion résultante. L'agent tensio-actif mis en œuvre est un copolymère polyéthylène-polyoxypropylène ayant un poids moléculaire compris entre 8 200 et 10 500, associé à la lécithine du jaune d'œuf ou à la lécithine des graines de soja. Ce procédé nécessite une opération de centrifugation et ne convient pas à tous les fluorocarbures.

Le brevet FR 74.03.244 (publié sous le n° 2 246 262) concerne une émulsion stable de composé fluorocarboné pouvant transporter de l'oxygène. Cette émulsion stable dans un milieu aqueux physiologiquement acceptable comprend : un composé perfluorocarboné, un phospholipide et au moins un composé d'acide gras comme adjuvant d'émulsification. Cette émulsion est préparée par mélange sous pression élevée.

La demande de brevet DE-OS 2.224.182 concerne un procédé pour la fabrication d'émulsion stable de particules de fluorocarbures ; ce procédé consiste à émulsionner sous pression élevée une solution dispersée contenant un fluorocarbure, un ou plusieurs agents tensio-actifs non ioniques et un ou plusieurs polyalcools pour former une émulsion de fluorocarbure ayant une dimension de particules inférieure à 0,2 µm.

D'autre part, le brevet US 3.778.381 concerne des microémulsions de fluorocarbures qui nécessitent pour leur fabrication la mise en œuvre d'un fluorohalogénocarbure à faible point d'ébullition. Elles sont obtenues par mélange d'un fluorocarbure avec un fluorohalogénocarbure, addition du mélange résultant à un système aqueux contenant un agent tensio-actif et agitation jusqu'à ce que la microémulsion soit formée, puis élimination dudit fluorohalogénocarbure par évaporation.

Le brevet FR 73.39.533 (publié sous le numéro 2.249.657) concerne un procédé de préparation de liquides à application biologique et transporteurs d'oxygène. Ce procédé consiste à émulsionner un composé fluoré en présence de deux émulsionnants dont l'un est à caractère hydrophile et l'autre à caractère lipophile. Les émulsionnants mis en œuvre dans ce procédé sont des agents tensio-actifs fluorés non ioniques du type polyoxyéthylène-fluoro-alkyl-éther.

Les brevets US 4.105.798 et FR 7.537.587 (publié sous le n° 2.313.024) concernent des émulsions aqueuses de composés polycycliques perfluorés, utilisables comme produits de remplacement du sang. Ces composés polycycliques comportent 9 à 18 atomes de carbone et au moins deux carbones tête de pont. Il ne s'agit pas de microémulsions.

La demande de brevet JP 73.36.514, publiée sous le n° 123.184/74 concerne l'obtention d'émulsions aqueuses contenant un dérivé fluorocarboné, comme constituant principal, un agent tensio-actif non ionique et de l'eau. La préparation de ces émulsions nécessite la mise en œuvre d'une part d'agents stabilisants d'émulsions, d'agents régulateurs de pH ou d'autres agents et d'autre part des moyens mécaniques, tels qu'agitateurs, homogénéisateurs et similaires. Il faut noter que les émulsions préparées selon le procédé décrit dans cette demande de brevet JP 73.36.514 sont stables au maximum pendant un mois, et à 25 °C comme le montrent les exemples illustratifs figurant dans cette demande de brevet JP. Par ailleurs on notera que les agents tensio-actifs mis en œuvre dans ce procédé sont obtenus, conformément aux procédés décrits dans cette demande de brevet JP sous la forme de mélanges ayant une répartition conforme à une courbe de Gauss et non pas sous la forme de produits purs.

Cet état de la technique montre que la fabrication de microémulsions de fluorocarbures est complexe et que les solutions proposées jusqu'à présent sont insatisfaisantes, soit qu'elles fassent appel à des sonications qui libèrent des ions fluor toxiques, soit qu'elles utilisent une homogénéisation mécanique qui nécessite de très grandes précautions pour obtenir des particules suffisamment fines, sans éliminer totalement les risques de libération d'ions fluor.

Il est, de plus, nécessaire d'utiliser des mélanges de divers tensio-actifs dont la composition n'est pas toujours connue avec précision ou de faire appel à un artifice de fabrication (par exemple selon le brevet US 3.778.381). Par ailleurs, ces émulsions sont instables et doivent être conservées congelées jusqu'au moment de l'utilisation.

Dans la thèse présentée à l'Université de NANCY I le 15 décembre 1978 par Monsieur MATHIS, il est indiqué que pour formuler des microémulsions aqueuses avec le minimum de tensio-actif non ionique, il faut choisir un agent tensio-actif fluoré non ionique possédant un point de trouble voisin de 37 °C pour réaliser des microémulsions de fluorocarbures utilisables comme substituts du sang. Contrairement aux enseignements de cette thèse qui laissait supposer que le comportement des systèmes ternaires eau/tensio-actifs fluorés non ioniques/fluorocarbures devait être identique à celui des systèmes ternaires contenant des composés hydrogénés homologues, on a trouvé que pour obtenir une microémulsion dans l'eau pure, stable à une température donnée que l'on peut choisir entre 5 °C et 90 °C, et ne contenant qu'un seul agent tensio-actif fluoré non ionique, il est indispensable d'utiliser un agent tensio-actif fluoré non ionique ayant un point de trouble nettement inférieur à ladite température désirée de stabilité de la microémulsion et plus particulièrement inférieur d'au moins 5 à 10 °C à cette température de stabilité.

On a maintenant trouvé de nouvelles microémultions qui peuvent être obtenues facilement par simple mélange de trois constituants définis ci-après et chauffage jusqu'à la température qui correspond à la température de stabilité de la microémulsion qui a été choisie.

La présente invention a donc pour objet des microémulsions aqueuses, formées par simple mélange, stables autour d'une température que l'on peut choisir entre 5 °C et 90 °C, composées :

a) d'un fluorocarbure
b) d'un agent tensioactif fluoré non ionique
c) et d'eau

caractérisées en ce que l'agent tensio-actif comporte un motif hydrophobe fluoré et une partie hydrophile non ionique et a un point de trouble inférieur d'au moins 5 à 10 °C à la température désirée de stabilité de la microémulsion et en ce que les proportions relatives des différents constituants sont choisies de façon telle que le point représentatif de la composition se trouve dans le domaine d'existence d'une microémulsion, tel qu'il apparaît sur le diagramme ternaire, tracé pour le mélange des 3 composés considérés, à ladite température de stabilité désirée.

Par « microémulsions » on désigne dans la suite de la description de la présente demande des solutions isotropes limpides et stables, composées d'une solution aqueuse d'agent tensioactif dans laquelle une huile est dispersée sous la forme de globules dont la taille peut varier de quelques nanomètres à quelques dizaines de nanomètres.

Le terme « milieu aqueux » désigne dans la présente description aussi bien l'eau pure que les solutés de remplissage et tous les milieux aqueux physiologiquement acceptables par exemple les solutions aqueuses salines ou les solutions aqueuses amylacées.

A titre de tels milieux, on peut citer notamment la solution physiologique de Ringer lactate et les solutions contenant de l'amidon soluble (H.E.S.) ou de la gélatine modifiée ainsi que le « Plasmion » (marque déposée). Dans les microémulsions selon l'invention, on peut utiliser un fluorocarbure quelconque c'est-à-dire un produit organique entièrement ou presque entièrement fluoré, non miscible à l'eau.

Toutefois, dans le cadre d'applications biologiques des microémulsions selon l'invention, c'est-à-dire à titre de transporteurs d'oxygène, il convient d'utiliser des composés fluorés, chimiquement inertes, non toxiques. D'autre part, pour ces applications biologiques, ces composés fluorés ne doivent pas ou pratiquement pas s'accumuler dans l'organisme ; ils doivent posséder une tension de vapeur suffisamment faible pour qu'ils n'entrent pas en ébullition dans l'organisme et avoir une aptitude importante à transporter l'oxygène, par exemple d'au moins 30 %.

A titre d'exemples de tels fluorocarbures, on pourra citer les fluorocarbures cités dans les brevets FR-A-2 105 287, A-2 218 903, A-2 246 262.

Parmi les fluorocarbures appropriés on citera plus particulièrement :

les perfluorocycloalcanes ou les perfluoroalkylcycloalcanes ;

les perfluoroalcanes et les fluoroalcanes partiellement hydrogénés saturés ou insaturés, tels que les produits de formules :

$$C_8F_{17}CH = CH_2 \; ; \; C_6F_{13}CH = CH_2 \text{ ou } C_8F_{17}C_2H_5 \; ;$$

les produits de formule générale $C_nF_{2n+1}-CH = CHC_mF_{2m+1}$ n et m étant des nombres entiers,

les composés hétérocycliques perfluorés alkyl-substitués, les amines tertiaires perfluorées, les éthers perfluorés.

A titre d'exemples préférés de tels fluorocarbures, on mentionnera les composés ci-après :

perfluorodécaline,
perfluorotributylamine,
perfluorotripropylamine,
perfluorométhyldécaline,

perfluoroadamantane,
perfluorodiméthyladamantane et
perfluorodécane.

A titre d'agents tensio-actifs fluorés non ioniques appropriés aux fins de l'invention, on citera les agents tensio-actifs du type polyoxyéthylène-fluoro-alkyléther.

Lorsque les microémulsions selon l'invention sont utilisées comme transporteurs d'oxygène dans l'organisme il importe de choisir des agents tensio-actifs fluorés non ioniques non toxiques et pharmaceutiquement compatibles et une température de stabilité voisine des températures atteintes par le corps humain, c'est-à-dire comprise entre environ 35 et 40 °C.

Une classe particulièrement préférée d'agents tensio-actifs du type polyoxyéthylène-fluoro-alkyléther est celle des composés répondant à la formule générale :

$$R_F(CH_2)\ (OC_2H_4)_nOH,$$

dans laquelle $R_F$ est une chaîne fluorocarbonée ou fluorocarbonée partiellement hydrogénée, n est un nombre entier au moins égal à 1.

Ces composés peuvent être obtenus par le procédé qui fait l'objet de la demande de brevet FR-A-2492 814 déposée le 24 octobre 1980 au nom du demandeur et intitulée « procédé pour la synthèse de composés hydrogénés et/ou fluorés du type polyoxyéthylène-alkyl-éther, composés de ce type et application à titre d'agents tensioactifs non ioniques ». Parmi ces composés, on citera tout particulièrement les composés de formules :

$C_7F_{15}CH_2(OC_2H_4)_4OH$ ; $C_7F_{15}CH_2\ (OC_2H_4)_5\ OH$ ; $C_7F_{15}CH_2\ (OC_2H_4)_6\ OH$ ; $C_6F_{13}CH_2\ (OC_2H_4)_3OH$ ; $C_6F_{13}CH_2\ (OC_2H_4)_4\ OH$ ; $C_6F_{13}CH_2(OC_2H_4)_5OH$ et $C_6F_{13}CH_2\ (OC_2H_4)_6OH$.

On a trouvé de façon surprenante que l'on pouvait obtenir une microémulsion stable à une température donnée en mélangeant simplement des quantités adéquates des trois constituants définis ci-dessus et en chauffant le mélange résultant jusqu'à la température de stabilité de la microémulsion désirée.

On a en effet trouvé qu'il existe des zones de concentrations relatives de ces trois constituants pour lesquelles on obtient, à une température donnée, une microémulsion stable. Si la température de la microémulsion obtenue est modifiée, par exemple au cours du transport ou lors de la conservation au réfrigérateur ou de la stérilisation, la microémulsion limpide se reforme de la même façon par agitation à la température de stabilité de la microémulsion considérée. cette microémulsion est stable indéfiniment dans le temps au voisinage de sa température de stabilité. On a également trouvé que pour un fluorocarbure donné la zone de stabilité de la microémulsion à une température donnée se déplace vers les fortes teneurs en fluorocarbure et les faibles teneurs en agent tensio-actif lorsque l'on diminue l'hydrophilie de l'agent tensio-actif. Il se produit un effet identique lorsque l'on ajoute des sels ou des macromolécules de type amidon soluble (H.E.S.), ou gélatine modifiée.

Il convient d'utiliser aux fins de l'invention, lorsque le milieu aqueux est de l'eau pure, un agent tensio-actif fluoré non ionique présentant un point de trouble inférieur à la température de stabilité de la micro-émulsion désirée et plus particulièrement inférieur d'au moins 5 à 10 °C à cette température de stabilité.

L'écart entre le point de trouble de l'agent tensio-actif et de la température de stabilité dépend d'une part du fluorocarbure, par exemple, dans le cas de fluorocarbures linéaires de même type, de la longueur de la chaîne fluorocarbonée et d'autre part, des différents additifs présents dans la phase aqueuse (sels, macromolécules).

Comme on l'a indiqué ci-dessus, cet écart doit être d'au moins 5 à 10 °C lorsque le milieu aqueux est constitué uniquement d'eau pure. Par contre, lorsqu'on ajoute des sels ou des macromolécules dans le milieu aqueux constitué d'eau pure, le tensio-actif, nécessaire pour obtenir une microémulsion occupant une position sensiblement comparable à celle de la microémulsion sans sel ni macromolécule (c'est-à-dire ayant sensiblement la même concentration en fluorocarbure et la même température de stabilité), doit être plus hydrophile (HLB plus élevé) c'est-à-dire avoir un point de trouble supérieur à celui du tensio-actif mis en œuvre pour obtenir la micro-émulsion dans de l'eau pure. Si on utilise le même agent tensio-actif dans les deux cas, la température de stabilité de la microémulsion contenant la même proportion de fluorocarbure et d'agent tensio-actif mais contenant plus de sels ou de macromolécules, sera, si la concentration en additifs est notable, inférieure à celle de la microémulsion formée avec de l'eau pure.

Il sera aisé à l'homme de l'art de déterminer, pour un fluorocarbure et un agent tensio-actif fluoré non ionique donnés, par des essais de routine, la température à laquelle se forme la microémulsion. Pour obtenir une microémulsion dudit fluorocarbure stable à une autre température, il y aura lieu de modifier les proportions relatives des trois constituants du mélange ou de choisir un agent tensioactif fluoré non ionique ayant une hydrophilie supérieure ou inférieure à celui utilisé pour la détermination ci-dessus, suivant que la température de stabilité de la microémulsion souhaitée est supérieure ou inférieure à celle définie ci-dessus.

A titre indicatif on précisera que lorsque le tensio-actif fluoré non ionique est du type polyoxyéthylène-fluoro-alkyl-éther, ayant un nombre bien défini de motifs éther, on peut obtenir des microémulsions

de perfluorodécaline, du fluorocarbure de formule $C_8F_{17}CH = CH_2$ ou de perfluorotributylamine stables à 37 °C et pouvant incorporer des quantités notables d'oxygène, en utilisant des agents tensio-actifs de ce type ayant un point de trouble inférieur ou voisin de 0 °C. Des exemples de tels tensio-actifs sont ceux ayant 6 à 7 atomes de carbone dans le radical $R_F$ et 5 ou 6 motifs éther.

Il importe alors de déterminer pour chaque fluorocarbure et pour un agent tensio-actif donné la zone de concentrations qui procure une microémulsion stable à une température donnée.

Il sera aisé à l'homme de l'art de déterminer ces zones par des essais de routine et d'établir pour chaque fluorocarbure le diagramme ternaire comportant ladite zone de concentration appropriée pour un agent tensio-actif donné.

Pour déterminer ces zones de concentration, on forme par exemple des mélanges binaires avec deux des trois constituants de la microémulsion et, à la température souhaitée de stabilité, on ajoute des quantités croissantes du troisième constituant et on note la quantité qui permet l'obtention de la microémulsion limpide ; cette quantité constitue la limite inférieure de la zone de concentration pour le système binaire considéré. On augmente ensuite la concentration du troisième constituant jusqu'à la démixtion pour fixer la limite supérieure de ladite zone. Ensuite, on fait varier la concentration relative du système binaire de départ et on opère de la même façon. Ce mode opératoire est répété un nombre suffisant de fois pour obtenir avec précision la zone de concentration appropriée et on établit ainsi le diagramme pour chaque système ternaire correspondant.

Les figures 1 à 4 annexées sont les diagrammes spécifiques pour les systèmes ci-après :

Figure 1 : eau/perfluorodécaline/$C_7F_{15}CH_2(OC_2H_4)_5OH$

Figure 2 : eau/$C_8F_{17}CH = CH_2$/$C_7F_{15}CH_2(OC_2H_4)_6OH$

Figure 3 : solution de Ringer lactée/$C_8F_{17}CH = CH_2$/$C_7F_{15}CH_2(OC_2H_4)_6OH$

Figure 4 : eau/perfluorodécaline/$C_6F_{13}CH_2(OC_2H_4)_5OH$

Ces diagrammes ont été déterminés selon le mode opératoire ci-dessus ; les zones hachurées constituent les zones de concentrations appropriées pour des températures de stabilité d'environ 37 °C et 25 °C respectivement.

Ainsi, comme on peut le voir sur la figure 1, poure obtenir une microémulsion stable à environ 37 °C avec la perfluorodécaline et l'agent tensio-actif de formule $C_7F_{15}CH_2(OC_2H_4)_5OH$ il faut utiliser en poids : environ 20 à 55 % de la perfluorodécaline, environ 2,5 à 15 % de l'agent tensio-actif $C_7F_{15}$-$CH_2(OC_2H_4)_5OH$, le complément étant de l'eau.

Pour obtenir une microémulsion stable à environ 37 °C avec le fluorocarbure de formule $C_8F_{17}CH = CH_2$ (voir figure 2) il faudra utiliser 0 à 60 % dudit fluorocarbure, 0 à 20 % de l'agent tensio actif $C_7F_{15}CH_2(OC_2H_4)_6OH$, le complément étant de l'eau.

Pour obtenir une microémulsion stable à environ 25 °C avec les mêmes constitutants, il faudra utiliser 0 à 45 % dudit fluorocarbure, 0 à 25 % de l'agent tensio-actif $C_7F_{15}CH_2(OC_2H_4)_6OH$, le complément étant de l'eau.

Pour obtenir une microémulsion stable à environ 37 °C avec le fluorocarbure de formule $C_8F_{17}CH = CH_2$, le milieu aqueux étant constitué de la solution lactée de Ringer il faudra utiliser (voir figure 3) :

0 à 55 % de $C_8F_{17}CH = CH_2$

0 à 20 % de l'agent tensioactif de formule $C_7F_{15}CH_2(OC_2H_4)_6OH$, le complément étant constitué de la solution de Ringer-lactate.

Pour obtenir une microémulsion stable à 37 °C de perfluorodécaline dans l'eau (voir figure 4) il convient d'utiliser :

0 à 45 % de perfluorodécaline

0 à 50 % de l'agent tensio-actif $C_6F_{13}CH_2$ $(OC_2H_4)_5OH$ le complément étant de l'eau.

Pour obtenir une microémulsion stable à 25 °C, il faudra utiliser pratiquement les mêmes gammes comme on peut le voir sur la figure 4.

Lorsque l'on a déterminé le diagramme ternaire d'un système eau/fluorocarbure/agent tensio-actif, à une température donnée, par exemple à 37 °C, il est alors possible de prévoir l'évolution de la position de la zone de la microémulsion, à cette même température, lorsque l'on modifie l'hydrophilie de l'agent tensio-actif mis en œuvre. On a trouvé en effet que lorsque l'on utilise un agent tensio-actif second plus hydrophile que l'agent actif premier, utilisé pour établir la zone de microémulsion d'un fluorocarbure donné à une température donnée, on obtient à la même température, une zone de microémulsion déplacée vers la base $H_2O$/agent tensio-actif du diagramme c'est-à-dire que le rapport massique fluorocarbure/agent tensio-actif second est plus faible que le rapport massique fluorocarbure/agent tensio-actif premier. Inversement, lorsqu'on utilise un agent tensio-actif second moins hydrophile que l'agent tensio-actif premier la zone de microémulsion s'éloigne de la base $H_2O$/agent tensio-actif.

A cet effet on peut se référer aux diagrammes des figures 1 et 4 ; pour le système ternaire $H_2O$/perfluorodécaline/$C_7F_{15}CH_2(OC_2H_4)_5OH$, la zone de microémulsion à 37 °C existe dans une gamme de composition au voisinage d'un rapport fluorocarbure/agent tensio-actif voisin de 80/20, soit 4 alors que pour le système ternaire $H_2O$/perfluorodécaline/$C_6F_{13}CH_2(OC_2H_4)_5OH$ la zone de microémulsion à 37 °C est au voisinage d'un rapport fluorocarbure/agent tensio-actif compris entre 65/35 et 60/40 soit de 1,85 à 1,50.

On notera que l'augmentation de l'hydrophilie d'un agent tensio-actif donné peut être obtenue soit

par augmentation du nombre de motifs éther, soit par diminution du nombre de motifs $CF_2$.

Comme on l'a indiqué précédemment la présence de sels ou de macromolécules modifie la position de la zone de microémulsion pour un système ternaire donné. On a trouvé que la présence de sels ou de macromolécules de type gélatine modifiée dans la phase aqueuse, a un effet comparable à celui engendré par une diminution de l'hydrophilie du tensio-actif, ce qui s'explique par un effet d'« effet de sel » ou de destructuration de l'eau bien connu pour les tensio-actifs non ioniques hydrogénés (voir à cet effet les références ci-après) :

— Friberg S. and Lapczynska I. Progr. Colloïd Polymer Sci. *56,* 16-20, 1975.

— Shinoda K., Takeda H., J. Coll. Interf. Sci. *32,* p. 642, 1970).

Ainsi, si l'on examine les microémulsions de perfluorodécaline obtenues à partir d'une solution de 20 % de $C_6F_{13}CH_2(OC_2H_4)_5OH$ dans l'eau ou le Ringer Lactate, on remarque que dans le premier cas, la microémulsion à 37 °C est obtenue pour des compositions de perfluorodécaline comprises entre 21 et 27 % alors que dans le deuxième cas elle se situe entre 24 et 29 % de perfluorodécaline, cet effet étant comparable à une diminution de l'hydrophilie du tensio-actif.

De la même façon, avec les microémulsions de perfluorodécaline obtenues à partir de solutions de 20 % de$C_7F_{15}CH_2(OC_2H_4)_6OH$ dans l'eau ou le « Plasmion », on remarque que dans le premier cas la microémulsion à 37 °C est obtenue pour des compositions en perfluorodécaline comprises entre 9 et 16 % alors que dans le deuxième cas, la microémulsion est obtenue pour des compositions en perfluorodécaline comprises entre 14 et 20 %, ce comportement étant encore une fois parallèle à celui qui serait engendré par une diminution de l'hydrophilie du tensio-actif. Ces résultats d'essais sont indiqués respectivement sur les diagrammes ternaires :

— eau ou Ringer-lactate/perfluorodécaline/$C_6F_{13}CH_2(OC_2H_4)_5OH$ de la figure 5 annexée et

— eau ou « Plasmion® »/perfluorodécaline/$C_7H_{15}CH_2(OC_2H_4)_6OH$ de la figure 6 annexée.

On notera que le produit connu sous la dénomination commerciale « Plasmion »® est un substitut du plasma sanguin constitué par l'addition d'une macromolécule (gélatine fluide modifiée) à un soluté ionique équilibré du type Ringer-lactate.

L'utilisation des émulsions de fluorocarbures selon l'art antérieur comme substituts du sang, nécessite le mélange de l'émulsion aqueuse de fluorocarbure avec des solutés de remplissage. Selon l'invention on peut obtenir des microémulsions prêtes à l'emploi, c'est-à-dire des microémulsions contenant le fluorocarbure comme transporteurs d'oxygène et le soluté de remplissage qui constitue le milieu aqueux utilisé pour la fabrication de la microémulsion. Il peut être avantageux de fabriquer des formulations pharmaceutiques constituant des unités de micro-émulsions de 300 ml par exemple, puisqu'il est courant d'utiliser dans les transfusions sanguines des unités de sang ou de substituts de sang de 300 ml. Ces formulations pharmaceutiques sont réalisées selon des moyens connus de l'homme de l'art pour satisfaire à la législation pharmaceutique. Des exemples d'unités de 300 ml seront donnés ci-après dans les exemples illustratifs.

La taille et la forme des particules des microémulsions selon l'invention peuvent être déterminées par diffusion des neutrons aux petits angles (Small angle neutron scattering). L'examen de la courbe de diffusion de la microémulsion permet de déterminer la valeur du nombre d'agrégation et du rayon de giration des particules à partir des expériences effectuées aux petits angles sur les microémulsions très diluées (région riche en eau).

La comparaison de la courbe obtenue aux plus grands angles avec un modèle théorique permet de déterminer la forme des particules.

Pour le système ternaire $H_2O/C_8F_{17}CH{=}CH_2/C_7F_{15}CH_2(OC_2H_4)_6OH$ représenté sur le diagramme de la figure 2 on a trouvé, qu'à la température de 25 °C pour un rapport massique fluorocarbure/agent tensioactif de 1,4 les particules étaient des éllipsoïdes possédant un nombre d'agrégation voisin 2 000 et un grand axe de 20 nm.

On a par ailleurs constaté que la taille et la forme des particules ne varient pas lors de la dilution de la microémulsion dans un milieu aqueux. D'autre part, on a constaté que la stabilité de la microémulsion n'est pas altérée lors de la dilution dans un milieu aqueux. Ainsi, lors de l'utilisation des microémulsions à titre de substituts du sang, leur stabilité et la taille des particules de celles-ci ne seront pas altérées dans l'organisme, puisqu'au cours de la transfusion de ces microémulsions il se produit une dilution de celles-ci dans le sang du patient. Des essais effectués avec des microémulsions selon le diagramme de la figure 2 ont en effet montré que, lors de la dilution en milieu aqueux de celles-ci, la dimension des particules n'était pas modifiée et que la stabilité de ces microémulsions était conservée.

La présente invention permet de pallier les difficultés de mise en émulsion et de mauvaise stabilité des solutions aqueuses de fluorocarbures utilisées jusqu'à présent.

Les microémulsions de l'invention trouvent une application particulière dans le domaine médical, notamment à titre de transporteur d'oxygène. Dans ce cas particulier, il importe seulement d'utiliser un agent tensio-actif non toxique chimiquement inerte, ne s'accumulant pas dans l'organisme.

Les microémulsions selon l'invention ont une capacité de fixation de l'oxygène meilleure que celle des émulsions de l'art antérieur, ce qui permet de travailler à des pressions d'oxygène plus faibles comparativement à celles nécessaires avec les émulsions de l'art antérieur, ces pressions d'oxygène plus faibles se rapprochant davantage des conditions physiologiques.

Les microémulsions selon l'invention sont également appropriées pour la perfusion d'organes isolés,

le transport d'azote ou de gaz carbonique ou comme additifs dans les milieux de cultures de bactéries aérobies, ou anaérobies.

L'invention va être maintenant décrite plus en détail dans les exemples illustratifs ci-après. Tous les pourcentages sont en poids aussi bien dans les exemples ci-après que sur les diagrammes des figures 1 à 6.

Exemple 1

Préparation d'une microémulsion

$H_2O/C_8F_{17}CH{=}CH_2/C_7F_{15}CH_2(OC_2H_4)_6OH$ stable à 37 °C.

On a déterminé la zone de concentrations spécifique de la stabilité à 37 °C du système ternaire ci-dessus en opérant selon le mode opératoire défini précédemment et on a établi le diagramme ternaire de la figure 2.

Dans un tube à essais on a ajouté :

3,5 g de $C_8F_{17}CHCH_2$

5,2 g d'eau

1,3 g de $C_7F_{15}CH_2(OC_2H_4)_6OH$ (point de trouble < 0 °C)

la solution a été portée à 37 °C dans un thermostat ; après agitation manuelle, on a obtenu une solution limpide stable indéfiniment à 37 °C.

On a refroidi la microémulsion ainsi obtenue jusqu'à 25 °C. Le mélange ternaire était alors sous la forme deux phases, une phase supérieure limpide, une phase inférieure laiteuse. On a ensuite porté ce mélange à 37 °C et on a obtenu à nouveau une microémulsion stable qui présentait les caractéristiques identiques à celles de la microémulsion obtenue après mélange des trois constituants et chauffage à 37 °C.

De la même manière on a mélangé dans un tube à essais :

4,5 g de $C_8F_{17}CHCH_2$

4 g d'eau

1,5 g de $C_7F_{15}CH_2(OC_2H_4)_6OH$

la solution a été portée à 37 °C dans un thermostat ; après une agitation manuelle, on a obtenu une solution limpide stable indéfiniment à 37 °C.

On a également obtenu une microémulsion stable à 37 °C en mélangeant selon le mode opératoire ci-dessus 2,5 g de $C_8F_{17}CHCH_2$, 6,5 g d'eau et 1 g de $C_7F_{15}CH_2(OC_2H_4)_6OH$. On a déterminé que cette microémulsion dissolvait 12 ml d'oxygène pour 100 ml de microémulsion à 37 °C.

Exemple 2

Préparation d'une microémulsion

Ringer lactate/$C_8F_{17}CH{=}CH_2/C_7F_{15}CH_2(OC_2H_4)_6OH$ stable à 37 °C.

On a déterminé la zone de concentration spécifique de stabilité à 37 °C du système ternaire ci-dessus en opérant selon le mode opératoire défini précédemment et on a établi une partie du diagramme ternaire représenté sur la figure 3.

Dans un tube à essai on a ajouté :

3,3 g $C_8F_{17}CHCH_2$

5,7 g de solution lactée de Ringer

1 g de $C_7F_{15}CH_2(OC_2H_4)_6OH$

le mélange a été porté à 37 °C ; après agitation manuelle on a obtenu une solution limpide stable indéfiniment à 37 °C.

Exemple 3

Préparation d'une microémulsion

eau/fluorodécaline/$C_7F_{15}CH_2(OC_2H_4)_5OH$.

On a déterminé la zone de concentration spécifique de la stabilité à 37 °C du système ternaire ci-dessus en opérant selon le mode opératoire défini précédemment et on a établi le diagramme ternaire de la figure 1.

Dans un tube à essai on a ajouté :

3,8 g de perfluorodécaline

5,3 g d'eau

0,9 g de $C_7F_{15}CH_2(OC_2H_4)_5OH$.

Le mélange a été porté à 37 °C ; après agitation manuelle, on a obtenu une solution limpide stable indéfiniment à 37 °C.

On a également obtenu une microémulsion indéfiniment stable à 37 °C en mélangeant :

4,16 g de perfluorodécaline
2,88 g d'eau
0,96 g de $C_7F_{15}CH_2(OC_2H_4)_5OH$.

On a déterminé que cette microémulsion dissolvait 17,5 ml d'oxygène pour 100 ml de microémulsion à 37 °C.

Exemple 4

Microémulsion système

$C_7F_{16}/H_2O/C_7F_{15}CH_2(OC_2H_4)_6OH$

On a utilisé un mélange constitué de :
1,3 g $C_7F_{15}CH_2(OC_2H_4)_6OH$
1,2 g $C_7F_{16}$
7,5 g $H_2O$.

On a opéré selon le mode opératoire défini précédemment et on a obtenu une microémulsion stable à 37 °C.

Exemple 5

Microémulsion système perfluorotributylamine/$H_2O/C_7F_{15}CH_2(OC_2H_4)_5OH$

On a utilisé un mélange constitué de :
1,8 g perfluorotributylamine
1,2 g $C_7F_{15}CH_2(OC_2H_4)_5OH$
7 g $H_2O$

On a obtenu une microémulsion stable à 37 °C.

Exemple 6

Microémulsion système perfluorodécaline/$H_2O/C_6F_{13}CH_2(OC_2H_4)_5OH$

On a utilisé un mélange constitué de :
2,5 g de perfluorodécaline
6 g d'eau
1,5 g de $C_6F_{13}CH_2(OC_2H_4)_5OH$.

Après avoir porté le mélange à 37 °C et agité manuellement on a obtenu une microémulsion indéfiniment stable.

Exemple 7

Système perfluorodécaline/Ringer lactate/$C_6F_{13}CH_2(OC_2H_4)_5OH$

On a utilisé un mélange constitué de :
2,9 g de perfluorodécaline
5,6 g de Ringer Lactate
1,5 g de $C_6F_{13}CH_2(OC_2H_4)_5OH$.

On a porté le mélange à 37 °C et après agitation manuelle, une microémulsion indéfiniment stable a été obtenue.

Exemple 8

Système perfluorodécaline/Plasmion/$C_7F_{15}CH_2(OC_2H_4)_6OH$

On a utilisé un mélange constitué de :
1,9 g de perfluorodécaline
6,5 g de Plasmion
1,6 g de $C_7F_{15}CH_2(OC_2H_4)_6OH$.

Après agitation manuelle du mélange à 37 °C, une microémulsion limpide indéfiniment stable à 37 °C a été obtenue.

Exemple 9

Système perfluorodécaline/$H_2O/C_7F_{15}CH_2(OC_2H_4)_6OH$

On a utilisé un mélange constitué de :
1,5 g de perfluorodécaline
6,7 g $H_2O$
1,8 g $C_7F_{15}CH_2(OC_2H_4)_6OH$.

Après agitation manuelle à 37 °C, une microémulsion limpide et indéfiniment stable à 37 °C a été obtenue.

Exemple 10

$C_8F_{17}CH_2CH_3$/Ringer Lactate/$C_7F_{15}CH_2(OC_2H_4)_6OH$

On a utilisé un mélange constitué de :
1,1 g $C_8F_{17}CH_2CH_3$
4,17 g Ringer Lactate
0,76 g $C_7F_{15}CH_2(OC_2H_4)_6OH$.

Après agitation manuelle à 37 °C, une microémulsion indéfiniment stable à 37 °C a été obtenue.

Exemple 11

Préparation d'unités de 300 ml

En opérant selon le mode opératoire défini dans l'exemple 1 on a établi les zones de concentrations appropriées pour obtenir des microémulsions indéfiniment stables avec les systèmes ternaires ci-après :
$C_8F_{17}CH{=}CH_2$/$C_7F_{15}CH_2(OC_2H_4)_6OH$/Ringer-Lactate
perfluorodécaline/$C_6F_{13}CH_2(OC_2H_4)_5OH$/Ringer-Lactate
perfluorodécaline/$C_7F_{15}CH_2(OC_2H_4)_6OH$/« Plasmion »
et on a formé les unités de 300 ml définies ci-après :

(1) 130,4 g de $C_8F_{17}CH{=}CH_2$
35,5 g de $C_7F_{15}CH_2(OC_2H_4)_6OH$.
La solution a été complétée à 300 ml avec du Ringer Lactate.

(2) 108 g de perfluorodécaline
55,9 g de $C_6F_{13}CH_2(OC_2H_4)_5OH$.
La solution a été complétée à 300 ml avec du Ringer Lactate.

(3) 56,5 g $C_7F_{15}CH_2(OC_2H_4)_6OH$
69,9 g perfluorodécaline.
La solution a été complétée à 300 ml avec du « Plasmion ».

Essais d'hémocompatibilité in vitro

On a recherché si les microémulsions selon l'invention avaient une action sur le sang humain en effectuant un thromboélastogramme, en mesurant le temps de thrombine diluée et en évaluant l'hémolyse.

A — Protocole d'essais :

le sang humain était du sang de sujets normaux, prélevé le jour de l'essai, en flacon siliconé, et sur citrate à 9 %, dans le rapport du 1/20 ;

les produits (microémulsions ou témoin) mis en présence de ce sang, en proportions variables, ont permis, après 10 minutes d'incubation à la température du laboratoire, de pratiquer un thromboélastogramme sur sang total, afin de mettre en évidence une action éventuelle sur la coagulation globale selon le mode opératoire décrit par C. RABY dans Biologie des Hémorragies et des Thromboses 1 vol., Masson et Cie, Edit. Paris 1966, p. 187 ;

un temps de thrombine diluée a été effectué sur les plasmas des sangs ayant été en contact avec les microémulsions selon l'invention pendant 30 minutes à la température du laboratoire selon le mode opératoire décrit par C. RABY à la page 119 de l'ouvrage cité ci-dessus.

L'hémolyse a été évaluée sur ces plasmas après centrifugation du sang à 2 000 g pendant 15 minutes.

B — Microémulsions testées et témoin

Dans ces essais on a utilisé les microémulsions selon l'invention définie ci-après :

Microémulsion R 716 C 81
tensio actif $C_7F_{15}CH_2(OC_2H_4)_6OH$ : 12,6 % en poids/poids
fluorocarbure $C_8F_{17}CH_2CH_3$ : 18,3 % en poids/poids
solution de Ringer Lactate : 69,1 % en poids/poids.

Microémulsion R 716 C 82
tensio actif $C_7F_{15}CH_2(OC_2H_4)_6OH$ : 9,80 % en poids/poids
Fluorocarbure : $C_8F_{17}CH{=}CH_2$ : 35,90 % en poids/poids
Solution de Ringer Lactate : 54,30 % en poids/poids.

Comme témoin on a utilisé l'agent tensio-actif seul EF 716 c'est-à-dire le produit de formule $C_7F_{15}CH_2(OC_2H_4)_6OH$, en solution dans de l'éthanol, puisque ce tensio-actif n'est pas soluble dans les solutés physiologiques. A des fins de comparaison, on a également utilisé les microémulsions ci-dessus d'une part en présence d'éthanol et d'autre part en l'absence d'éthanol.

Dans la détermination du temps de thrombine on a utilisé de l'eau physiologique comme témoin.

C — Résultats :

En présence d'éthanol (V/V), une solution limpide a été obtenue avec l'agent tensio-actif. Celle-ci, ajoutée dans les proportions de 0,2 ml à 1,8 ml d'eau physiologique, a donné, après agitation, une émulsion laiteuse, instable, s'éclaircissant en 15 minutes environ, tant à la température du laboratoire qu'à 37 °C.

Cette émulsion, mise en présence de quatre échantillons de sang humain, à raison de 0,1 ml pour 0,9 ml de sang, a permis d'effectuer les essais définis ci-dessus.

Aucune action directe n'a été observée après adjonction du tensio-actif $C_7F_{15}CH_2(OC_2H_4)_6OH$ à du sang normal. La fluidité n'a pas été altérée, et il n'existait aucune trace d'hémolyse.

Après recalcification, déclenchant la coagulation du sang total, et dont les phénomènes sont enregistrés par le thromboélastogramme, on a constaté un léger raccourcissement de la constante « r », un allongement de « k » et une diminution de l'amplitude « amx ». Ces modifications mineures n'ont pas de signification particulière.

Microémulsions R 716 C 81 — R 716 C 82

1) En présence d'éthanol

Des essais similaires à ceux pratiqués avec l'agent tensio-actif en présence d'éthanol, ont donné des résultats identiques.

2) Sans éthanol

Ces émulsions, troubles à la température du laboratoire, ont donné des microémulsions limpides lorsqu'elles ont été portées quelques instants à 37 °C.

Utilisées pures ou diluées au 1/10 en eau physiologique, sans adjonction d'éthanol, et mises en présence de sang humain dans les mêmes proportions que précédemment et pendant un laps de temps identique, R 716 C 81 et R 716 C 82 ont produit les mêmes effets que ceux déjà observés avec éthanol.

Le temps de thrombine diluée, pratiqué dans cet essai, est très légèrement allongé par rapport au témoin constitué dans cet essai par de l'eau physiologique, le R 716 C 82 produisant les temps les plus longs. Ces temps de thrombine diluée sont indiqués dans le tableau I ci-après.

Tableau I
Temps de thrombine

| | Sang I | Sang II |
|---|---|---|
| Témoin eau physiologique | 26 s | 28 s |
| R 716 C 81 non dilué | 28 s | 31 s |
| R 716 C 81 dilué 1/10 | 28 s | 29 s |
| R 716 C 82 non dilué | 32 s | 33 s |
| R 716 C 82 dilué 1/10 | 31 s | 32 s |

Ces essais montrent que l'agent tensio-actif seul et les microémulsions R 716 C 81 et R 716 C 82 sont sans effet in vitro sur le sang humain, non coagulé, ne produisent pas d'activité semblable à la thrombine et ne semblent pas apporter de modifications au niveau de la membrane érythrocytaire, étant donné l'absence d'hémolyse.

**Revendications** (pour les Etats contractants : CH, BE, DE, FR, GB, IT, LU, NL, SE, LI)

1. Microémulsion aqueuse, formée par simple mélange, stable autour d'une température que l'on peut choisir entre 5 °C et 90 °C, composée :

a) d'un fluorocarbure
b) d'un agent tensio-actif fluoré, non ionique,
c) et d'eau,

caractérisée en ce que l'agent tensio-actif comporte un motif hydrophobe fluoré et une partie hydrophile non ionique et a un point de trouble de 5 à 10 °C inférieur à la température de stabilité désirée, et en ce que les proportions relatives des différents constituants sont choisies de façon telle que le point représentatif de la composition se trouve dans le domaine d'existence d'une microémulsion tel qu'il apparaît sur le diagramme ternaire, tracé pour le mélange des 3 composés considérés, à ladite température de stabilité désirée.

2. Microémulsion selon la revendication 1, caractérisée en ce que l'eau est remplacée par un milieu aqueux physiologiquement acceptable, une solution aqueuse saline ou amylacée.

3. Microémulsion selon l'une des revendications 1 ou 2, caractérisée en ce que le fluorocarbure est un perfluorocycloalcane, un perfluoroalkylcycloalcane, un perfluoroalcane, ou un fluoroalcane partiellement hydrogéné saturé ou insaturé, un produit de formule $C_nF_{2n+1}CH=CH—C_mF_{2m+1}$, n et m étant des nombres entiers : un composé hétérocyclique perfluoré alkyl-substitué, une amine tertiaire perfluorée, un éther perfluoré.

4. Microémulsion selon l'une quelconque des revendications 1 à 3, caractérisée en ce que le fluorocarbure est choisi parmi les composés ci-après :

perfluorodécaline,
perfluorotributylamine,
perfluorotripropylamine,
perfluorométhyldécaline,
perfluoroadamantane,
perfluorodiméthyladamantane, et
perfluorodécane, le composé de formule $C_8F_{17}CH=CH_2$ ou le composé de formule $C_8F_{17}CH_2CH_3$.

5. Microémulsion selon l'une quelconque des revendications 1 à 4, caractérisée en ce que l'agent tensi-actif est un composé de formule $R_F—CH_2—(OC_2CH_4)_n—OH$ dans laquelle $R_F$ est une chaîne fluorocarbonée et n est supérieur ou égal à 1.

6. Microémulsion selon la revendication 5, caractérisée en ce que l'agent tensioactif est choisi parmi :

$C_7F_{15}CH_2(OC_2H_4)_4OH$ ; $C_7F_{15}CH_2(OC_2H_4)_5OH$ ;
$C_7F_{15}CH_2(OC_2H_4)_6OH$ ; $C_6F_{13}CH_2(OC_2H_4)_3OH$ ;
$C_6F_{13}CH_2—(OC_2H_4)_4OH$ ;
$C_6F_{13}CH_2(OC_2H_4)_5OH$ et $C_6F_{13}CH_2(OC_2H_4)_6OH$.

7. Microémulsion selon l'une des revendications 1 à 6, stable autour de 37 °C, caractérisée en ce qu'elle comprend :

de 20 à 55 % en poids de perfluorodécaline
de 2 à 15 % en poids de $C_7F_{15}CH_2(OC_2H_4)_5OH$
et de l'eau

en proportions relatives définies par le diagramme ternaire d'émulsification à 37 °C.

8. Microémulsion selon l'une des revendications 1 à 6 stable autour de 37 °C, caractérisée en ce qu'elle comprend :

de 1 à 55 % en poids $C_8F_{17}CH=CH_2$
de 1 à 18 % en poids de $C_7F_{15}CH_2(OC_2H_4)_6OH$
et de l'eau,

en proportions relatives définies par le diagramme ternaire d'émulsification à 37 °C.

9. Microémulsion selon l'une des revendications 1 à 6, stable autour de 37 °C, caractérisée en ce qu'elle comprend :

de 25 à 50 % en poids de $C_8F_{17}CH=CH_2$
de 7 à 15 % en poids de $C_7F_{15}CH_2(OC_2H_4)_6OH$
et de la solution de Ringer Lactate

en proportions relatives définies par le diagramme ternaire d'émulsification à 37 °C.

10. Microémulsion selon l'une des revendications 1 à 6, stable autour de 37 °C, caractérisée en ce qu'elle comprend :

de 1 à 35 % en poids de perfluorodécaline
de 1 à 20 % en poids de $C_6F_{13}CH_2(OC_2H_4)_5OH$
et de l'eau

en proportions relatives définies par le diagramme ternaire d'émulsification à 37 °C.

11. Microémulsion selon l'une des revendications 1 à 6, stable autour de 25 °C, caractérisée en ce qu'elle comprend :

de 1 à 30 % de perfluorodécaline

de 1 à 30 % de $C_6F_{13}CH_2(OC_2H_4)_5OH$
et de l'eau
en proportions relatives définies par le diagramme ternaire d'émulsification à 25 °C.

12. Application des microémulsions selon l'une quelconque des revendications 1 à 11, contenant une quantité suffisante de fluorocarbure comme agents transporteurs des gaz tels que $O_2$, $N_2$, $CO_2$, comme agents thérapeutiques ou pour la perfusion d'organes isolés ou comme additifs dans les milieux de culture de bactéries.

13. Composition pharmaceutique caractérisée en ce qu'elle comprend une microémulsion selon l'une quelconque des revendications 1 à 10, contenant une quantité suffisante de fluorocarbure pour le transport des gaz du sang.

**Revendications** (pour l'Etat contractant AT)

1. Procédé de préparation d'une microémulsion aqueuse, stable autour d'une température que l'on peut choisir entre 5° et 90 °C, et composée
a) d'un fluorocarbure,
b) d'un agent tensio-actif fluoré, non ionique
c) d'eau
caractérisé en ce qu'on utilise un agent tensio-actif comportant un motif hydrophobe fluoré et une partie hydrophile non ionique et qui possède un point de trouble de 5 à 10 °C inférieur à la température de stabilité désirée et en ce qu'on mélange les 3 constituants en des proportions relatives qui sont celles relevées dans le domaine d'existence de la microémulsion qui a été préalablement déterminé sur le diagramme ternaire pour l'ensemble des 3 constituants à ladite température.

2. Procédé de préparation selon la revendication 1, caractérisé en ce que l'eau est remplacée par un milieu aqueux physiologiquement acceptable, une solution aqueuse saline ou amylacée.

3. Procédé selon l'une des revendications 1 ou 2, caractérisé en ce que le fluorocarbure est choisi parmi les perfluorocycloalcanes, les perfluoroalkylcycloalcanes, les perfluoroalcanes, ou les fluoroalcanes partiellement hydrogénés, saturés ou non, les produits de formule $C_nF_{2n+1}CH = CH—C_mF_{2m+1}$ (n et m étant des nombres entiers), les composés hétérocycles perfluorés alkylsubstitués, les amines tertiaires perfluorées et les éthers perfluorés.

4. Procédé selon l'une des revendications 1 à 3, caractérisé en ce que le fluorocarbure est choisi parmi :
la perfluorodécaline,
la perfluorotributylamine,
la perfluorotripropylamine,
la perfluorométhyldécaline,
le perfluoroadamantane,
le perfluorodiméthyladamantane, et
le perfluorodécane, le composé de formule $C_8F_{17}CH = CH_2$ ou le composé de formule $C_8F_{17}CH_2$-$CH_3$.

5. Procédé selon l'une des revendications 1 à 4 caractérisé en ce que l'agent tensio-actif est un composé de formule $R_F—CH_2—(OC_2CH_4)_n—OH$ dans laquelle $R_F$ est une chaîne fluorocarbonée et n est supérieur ou égal à 1.

6. Procédé selon la revendication 5, caractérisé en ce que l'agent tensioactif est choisi parmi :
$C_7F_{15}CH_2(OC_2H_4)_4OH$ ; $C_7F_{15}CH_2(OC_2H_4)_5OH$ ;
$C_7F_{15}CH_2(OC_2H_4)_6OH$ ; $C_6F_{13}CH_2(OC_2H_4)_3OH$ ;
$C_6F_{13}CH_2—(OC_2H_4)_4OH$ ;
$C_6F_{13}CH_2(OC_2H_4)_5OH$ et $C_6F_{13}CH_2(OC_2H_4)_6OH$.

7. Procédé selon l'une des revendications 1 à 6, caractérisé en ce que la microémulsion, stable autour de 37 °C est préparée par simple mélange de :
20 à 55 % en poids de perfluorodécaline
2 à 15 % en poids de $C_7F_{15}CH_2(OC_2H_4)_5OH$
et d'eau
en proportions relatives définies par le diagramme ternaire d'émulsification à 37 °C.

8. Procédé selon l'une des revendications 1 à 6, caractérisé en ce que la microémulsion, stable autour de 37 °C est préparée par simple mélange de :
1 à 55 % en poids $C_8F_{17}CH = CH_2$
1 à 18 % en poids de $C_7F_{15}CH_2(OC_2H_4)_6OH$
et d'eau,
en proportions relatives définies par le diagramme ternaire d'émulsification à 37 °C.

9. Procédé selon l'une des revendications 1 à 6, caractérisé en ce que la microémulsion, stable autour de 37 °C est préparée par simple mélange de :
25 à 50 % en poids de $C_8F_{17}CH = CH_2$

7 à 15 % en poids de $C_7F_{15}CH_2(OC_2H_4)_6OH$
et de la solution de Ringer lactate
en proportions relatives définies par le diagramme ternaire d'émulsification à 37 °C.

10. Procédé selon l'une des revendications 1 à 6, caractérisé en ce que la microémulsion stable autour de 37 °C est préparée par simple mélange de :
1 à 35 % en poids de perfluorodécaline
1 à 20 % en poids de $C_6F_{13}CH_2(OC_2H_4)_5OH$
et d'eau
en proportions relatives définies par le diagramme ternaire d'émulsification à 37 °C.

11. Procédé selon l'une des revendications 1 à 6, caractérisé en ce que la microémulsion stable autour de 25 °C est préparée par simple mélange de :
1 à 30 % de perfluorodécaline
1 à 30 % de $C_6F_{13}CH_2(OC_2H_4)_5OH$
et d'eau
en proportions relatives définies par le diagramme ternaire d'émulsification à 25 °C.

12. Procédé de maintien d'organes en survie, caractérisé en ce qu'on les perfuse avec une microémulsion préparée selon l'une quelconque des revendications 1 à 11, agissant comme transporteur des gaz tels que $O_2$, $N_2$, $CO_2$.

13. Procédé de préparation d'une solution de transport des gaz du sang, caractérisé en ce que la microémulsion contient une quantité suffisante de perfluorocarbure pour ce transport.

**Claims** (for the Contracting States : BE, CH, DE, FR, GB, IT, LI, LU, NL, SE)

1. An aqueous microemulsion, obtained by gentle mixing, stable around a temperature which can be selected from 5 °C to 90 °C, said microemulsion comprising :
a) a fluorocarbon
b) a nonionic fluorinated surfactant
c) water
and characterized in that the surfactant comprises a hydrophobic fluoro moiety and a hydrophylic nonionic portion, and has a cloud point which in from 5 °C to 10 °C less than the desired stability temperature, wherein the relative amounts of the components are selected so that representative point for said composition is located in the region corresponding to formation of microemulsion on the triangular three-component phase diagram, previously plotted for the mixture of the three compounds at said desired stability temperature.

2. A microemulsion as claimed in claim 1, characterized in that water is substituted by an aqueous physiologicaly acceptable medium, a salted or starch aqueous solution.

3. A microemulsion as claimed in claim 1 or 2, characterized in that said fluorocarbon is a perfluorocycloalkane, a perfluoroalkylcycloalkane, a perfluoroalkane, or a saturated or unsaturated partially hydrogenated fluoroalkane, a compound of formula $C_nH_{2n+1}—CH = CH—C_mF_{2m+1}$, wherein m and n are integers ; an alkyl substituted perfluoro heterocyclic compound, a perfluoro tertiary amine, a perfluoro ether oxyde.

4. A microemulsion as claimed in any claim 1 to 3, characterized in that said fluorocarbon is selected from the following compounds :
perfluorodecalin,
perfluorotributylamine,
perfluorotripropylamine,
perfluoromethyldecalin,
perfluoroadamantane,
perfluorodimethyladamantane
and perfluorodecane, the compound of the formula $C_8F_{17}CH = CH_2$ or the compound of the formula $C_8F_{17}CH = CH_2$ or the compound of the formula $C_8F_{17}CH_2CH_3$.

5. A microemulsion as claimed in claim 1 to 4 characterized in that said surfactant is a compound of formula $R_F—CH_2—(OC_2H_4)_n—OH$ wherein $R_F$ is a fluorocarbon chain and n has a value higher or equal to 1.

6. A microemulsion as claimed in claim 5 characterized in that the surfactant is selected from
$C_7F_{15}CH_2(OC_2H_4)_4OH$ ; $C_7F_{15}CH_2(OC_2H_4)_5OH$ ;
$C_7F_{15}CH_2(OC_2H_4)_6OH$ ; $C_6F_{13}CH_2(OC_2H_4)_3OH$ ;
$C_6F_{13}CH_2(OC_2H_4)_4OH$ ; $C_6F_{13}CH_2(OC_2H_4)_5OH$ and
$C_6F_{13}CH_2(OC_2H_4)_6OH$.

7. A microemulsion as claimed in any claim 1 to 6, stable around 37 °C, characterized in that it comprises
from 20 to 55 wt% perfluorodecalin,
from 2 to 15 wt% $C_7F_{15}CH_2(OC_2H_4)_5OH$
and water

in relative amounts determined on the three-component emulsification phase diagram. at 37 °C.

8. A microemulsion as claimed in any claim 1 to 6, stable around 37 °C, characterized in that it comprises

from 1 to 55 wt% $C_8F_{17}CH = CH_2$,
from 1 to 18 wt% $C_7F_{15}CH_2(OC_2H_4)_6OH$
and water

in relative amounts determined on the three-component emulsification phase diagram at 37 °C.

9. A microemulsion as claimed in any claim 1 to 6, stable around 37 °C, characterized in that it comprises

from 25 to 50 wt% $C_8F_{17}CH = CH_2$,
from 7 to 15 wt% $C_7F_{15}CH_2(OC_2H_4)_6OH$
and Ringer lactate medium

in relative amounts determined on the three-component emulsification phase diagram at 37 °C.

10. A microemulsion as claimed in any claim 1 to 6, stable around 37 °C, characterized in that it comprises

from 1 to 35 wt% perfluorodecalin,
from 1 to 20 wt% $C_6F_{13}CH_2(OC_2H_4)_5OH$
and water

in relative amounts determined on the three-component emulsification phase diagram at 37 °C.

11. A microemulsion as claimed in any claim 1 to 6, stable around 25 °C, characterized in that it comprises

from 1 to 30% perfluorodecalin,
from 1 to 30% $C_6F_{13}CH_2(OC_2H_4)_5OH$
and water

in relative amounts determined on the three-component emulsification phase diagram at 25 °C.

12. Use of microemulsions according to any claim 1 to 11, comprising an effective amount of fluorocarbon as gas carriers, for gas such as $O_2$, $N_2$, $CO_2$, useful as therapeutical agents or for perfusion of isolated organs or as additives in bacterium culture media.

13. A pharmaceutical composition characterized in that it comprises a microemulsion according to any claim 1 to 10, comprising an effective amount of fluorocarbon for blood gas carrying.

**Claims** (for the Contracting State AT)

1. Process for producing an aqueous microemulsion stable around a temperature which can be selected from 5 °C to 90 °C, which comprises :

a) a fluorocarbon
b) a nonionic fluorinated surfactant
c) water

characterized in that the surfactant comprises a fluoro hydrophobic moiety and a nonionic hydrophilic portion and has a cloud point which is 5 °C to 10 °C less than the desired stability temperature wherein the three components are present in relative amounts which are determined by the microemulsion region on the triangular three-component phase diagram, plotted previously for said temperature.

2. Process as claimed in claim 1 characterized in that an aqueous physiologically acceptable medium, a salted or starch aqueous solution are used as substitute for water.

3. Process as claimed in claim 1 or 2, characterized in that the florocarbon is selected from perfluorocycloalkanes, perfluoroalkylcycloalkanes, perfluoroalkanes or partially hydrogenated fluoroalkanes, saturated or unsaturated, compounds having the formula $C_nF_{2n+1}—CH = CH—C_mF_{2m+1}$, where n and m are integers, alkylsubstituted perfluoro heterocyclic compounds, perfluoro tertiary amines, and perfluoroether oxydes.

4. Process as claimed in claim 1 to 3, characterized in that the fluorocabon is selected from :

perfluorodecalin
perfluorotributylamine
perfluorotripropylamine
perfluoromethyldecalin
perfluoroadamantane
perfluorodimethyladamantane, and perfluorodecane, the compound having the formula $C_8F_{17}—CH = CH_2$ or the compound having the formula $C_8F_{17}CH_2CH_3$.

5. Process as claimed in claim 1 to 4, charcterized in that the surfactant is a compound having the formula $R_F—CH_2—(OC_2H_4)_n—OH$, wherein $R_F$ is a fluorocarbon chain and n has a value higher or equal to 1.

6. Process as claimed in claim 5, characterized in that the surfactant is selected from :

$C_7F_{15}CH_2(OC_2H_4)_4OH$ ; $C_7F_{15}CH_2(OC_2H_4)_5OH$ ;
$C_7H_{15}CH_2(OC_2H_4)_6OH$ ; $C_6F_{13}CH_2(OC_2H_4)_3OH$ ;
$C_6F_{13}CH_2(OC_2H_4)_4OH$ ; $C_6F_{13}CH_2(OC_2H_4)_5OH$ and
$C_6F_{13}CH_2(OC_2H_4)_6OH$

7. Process as claimed in any claim 1 to 6, characterized in that a microemulsion, stable around 37 °C, is produced by gentle mixing
from 20 to 55 w% perfluorodecalin,
from 2 to 15 wt% $C_7F_{15}CH_2(OC_2H_4)\cdot OH$ and water
in relative amounts determined on the three-component emulsification phase diagram at 37 °C.

8. Process as claimed in any claim 1 to 6, characterized in that a microemulsion, stable around 37 °C, is produced by gentle mixing
from 1 to 55 wt% $C_8F_{17}CH = CH_2$,
from 1 to 18 wt% $C_7F_{15}CH_2(OC_2H_4)_6OH$ and water
in relative amounts determined on the three component emulsification phase diagram at 37 °C.

9. Process as claimed in any claim 1 to 6, characterized in that a microemulsion, stable around 37 °C, is produced by gentle mixing
from 25 to 50 wt% $C_8F_{17}CH = CH_2$,
from 7 to 15 wt% $C_7F_{15}CH_2(OC_2H_4)_6OH$
and Ringer lactate medium in relative amounts determined on the three component emulsification phase diagram, at 37 °C.

10. Process as claimed in any claim 1 to 6, characterized in that a microemulsion, stable around 37 °C, is produced by gentle mixing
from 1 to 35 wt% perfluorodecalin,
from 1 to 20 wt% $C_6F_{13}CH_2(OC_2H_4)_5OH$
and water
in relative amounts determined on the three component emulsification phase diagram at 37 °C.

11. Process as claimed in any claim 1 to 6, characterized in that a microemulsion, stable around 25 °C, is produced by gentle mixing
from 1 to 30 wt% perfluorodecalin,
from 1 to 30 wt% $C_6F_{13}CH_2(OC_2H_4)_5OH$
and water
in relative amounts determined on the three-component emulsifcation phase diagram at 25 °C.

12. Process for keeping organs alive, characterized in that they are perfused with a microemulsion produced by process claimed in any claim 1 to 11, which works as a carrier of gas, such as $O_2$, $N_2$, $CO_2$.

13. Process for preparing a solute for carrying blood gas characterized in that the microemulsion contains an effective amount of perfluorocarbon for said carriage.

**Patentansprüche** (für die Vertragsstaaten : CH, BE, DE, FR, GB, IT, LU, NL, SE, LI)

1. Durch einfaches Mischen gebildete wässrige Mikroemulsion, die um eine Temperatur, ausgewählt von 5 bis 90 °C, stabil ist und aus
a) einem Fluorkohlenstoff,
b) einem fluorierten, nicht-ionischen, oberflächenaktiven Mittel und
c) Wasser
besteht, dadurch gekennzeichnet, daß das oberflächenaktive Mittel einen hydrophoben fluorierten Teil und einen hydrophilen, nicht-ionischen Teil sowie einen Trübungspunkt hat, der 5 bis 10 oC unter der gewünschten Stabilitätstemperatur liegt, und daß die relativen Verhältnisse der verschiedenen Bestandteile so ausgewählt sind, daß sich der repräsentative Punkt der Zusammensetzung im Bereich des Vorliegens einer Mikroemulsion, wie er auf dem ternären, für die Mischung der drei in betracht kommenden Verbindungen bei der gewünschten Stabilitätstemperatur erscheint, befindet.

2. Mikroemulsion nach Anspruch 1, dadurch gekennzeichnet, daß das Wasser durch ein physiologisch annehmbares wässriges Medium, eine wässrige Kochsalzlösung oder Stärkelösung ersetzt wird.

3. Mikroemulsion nach einem der Ansprüche 1 oder 2, dadurch gekennzeichnet, daß der Fluorkohlenstoff ein Perfluorcycloalkan, ein Perfluoralkylcycloalkan, ein Perfluoralkan oder ein teilweise hydriertes, gesättigtes oder ungesättigtes Fluoralkan, ein Produkt der Formel $C_nF_{2n+1}CH = CH—C_mF_{2m+1}$, wobei n und m ganze Zahlen sind, eine heterocyclische perfluorierte alkylsubstituierte Verbindung, ein tertiäres perfluoriertes Amin oder ein perfluorierter Ether ist.

4. Mikroemulsion nach einem der Ansprüche 1 bis 3, dadurch gekennzeichnet, daß der Fluorkohlenstoff aus den folgenden Verbindungen ausgewählt ist :
Perfluordecalin,
Perfluortributylamin,
Perfluortripropylamin,
Perfluormethyldecalin,
Perfluoradamantan,
Perfluordimethyladamantan
und Perfluordecan, der Verbindung der Formel $C_8F_{17} = CH_2$ oder der Verbindung der Formel $C_8F_{17}CH_2CH_3$.

5. Mikroemulsion nach einem der Ansprüche 1 bis 4, dadurch gekennzeichnet, daß das oberflächenaktive Mittel eine Verbindung der Formel $R_F$—$CH_2$—$(OC_2H_4)_n$—OH ist, in welcher $R_F$ eine Fluorkohlenstoffkette ist und n größer als oder gleich 1 ist.

6. Mikroemulsion nach Anspruch 5, dadurch gekennzeichnet, daß das oberflächenaktive Mittel ausgewählt ist aus:

$C_7F_{15}CH_2(OC_2H_4)_4OH$ ; $C_7F_{15}CH_2(OC_2H_4)_5OH$ ;
$C_7H_{15}CH_2(OC_2H_4)_6OH$ ; $C_6H_{13}CH_2(OC_2H_4)_3OH$ ;
$C_6F_{13}CH_2(OC_2H_4)_4OH$ ; $C_6F_{13}CH_2(OC_2H_4)_5OH$ und
$C_6F_{13}CH_2(OC_2H_4)_6OH$.

7. Mikroemulsion nach einem der Ansprüche 1 bis 6, die um 37 °C stabil ist, dadurch gekennzeichnet, daß sie

20 bis 55 Gew.-% Perflurdecalin,
2 bis 15 Gew.-% $C_7F_{15}CH_2(OC_2H_4)_5OH$ und
Wasser

in den durch das ternäre Emulgierungsdiagramm bei 37 °C definierten relativen Verhältnissen enthält.

8. Mikroemulsion nach einem der Ansprüche 1 bis 6, die um 37 °C stabil ist, dadurch gekennzeichnet, daß sie

1 bis 55 Gew.-% $C_8F_{17}CH = CH_2$,
1 bis 18 Gew.-% $C_7F_{15}CH_2(OC_2H_4)_6OH$ und
Wasser

in den durch das ternäre Emulgierungsdiagramm bei 37 °C definierten relativen Verhältnissen enthält.

9. Mikroemulsion nach einem der Ansprüche 1 bis 6, die um 37 °C stabil ist, dadurch gekennzeichnet, daß sie

25 bis 50 Gew.-% $C_8F_{17}CH = CH_2$,
7 bis 15 Gew.-% $C_7F_{15}CH_2(OC_2H_4)_6OH$ und
eine Ringer-Lactatlösung

in den durch das ternäre Emulgierungsdiagramm bei 37 °C definierten relativen Verhältnissen enthält.

10. Mikroemulsion nach einem der Ansprüche 1 bis 6, die um 37 °C stabil ist, dadurch gekennzeichnet, daß sie

1 bis 35 Gew.-% Perfluordecalin,
1 bis 20 Gew.-% $C_6F_{13}CH_2(OC_2H_4)_5OH$ und
Wasser

in den durch das ternäre Emulgierungsdiagramm bei 37 °C definierten relativen Verhältnissen enthält.

11. Mikroemulsion nach einem der Ansprüche 1 bis 6, die um 25 °C stabil ist, dadurch gekennzeichnet, daß sie

1 bis 30 Gew.-% Perfluordecalin,
1 bis 30 Gew.-% $C_6F_{13}CH_2(OC_2H_4)_5OH$ und
Wasser

in den durch das ternäre Emulgierungsdiagramm bei 25 °C definierten relaven Verhältnissen enthält.

12. Die Verwendung der Mikroemulsionen nach einem der Ansprüche 1 bis 11,, die eine ausreichende Menge Fluorkohlenstoff enthält, als Transportmittel für Gase, wie $O_2$, $N_2$, $CO_2$, als therapeutische Mittel oder zum Durchströmen isolierter Organe oder als Zusätze in Bakterienkulturmedien.

13. Pharmazeutische Zusammensetzung, dadurch gekennzeichnet, daß sie eine Mikroemulsion nach einem der Ansprüche 1 bis 10 umfaßt, die eine ausreichende Menge Fluorkohlenstoff zum Transport von Blutgasen enthält.

**Patentansprüche** (für den Vertragsstaat AT)

1. Verfahren zur Herstellung einer wässrigen Mikroemulsion, die bei einer Temperatur, die man von 5 bis 90 °C wählen kann, stabil ist und aus

a) einem Fluorkohlenstoff
b) einem fluorierten, nicht-ionischen oberflächenaktiven Mittel und
c) Wasser

besteht, dadurch gekennzeichnet, daß man ein oberflächenaktives Mittel verwendet, das einen hydrophoben fluorierten Teil und einen hydrophilen nicht-ionischen Teil aufweist und einen Trübungspunkt bestitzt, der 5 bis 10 °C unter der gewünschten Stabilitätstemperatur liegt, und daß man die drei Bestandteile in relativen Verahältnissen mischt, die im Bereich des Vorliegens der Mikroemulsion, das vorher auf einem ternären Diagramm durch die Gesamtheit der drei Bestandteile bei der genannten Temperatur bestimmt wurde, liegen.

2. Verfahren zur Herstellung gemäß Anspruch 1, dadurch gekennzeichnet, daß das Wasser durch ein physiologisch annehmbares wässriges Medium, eine wässrige Kochlsalzlösung oder Stärkelösung ersetzt wird.

3. Verfahren nach einem der Ansprüche 1 oder 2, dadurch gekennzeichnet, daß der Fluorkohlenstoff ausgewählt ist aus Perfluorcycloalkanen, Perfluoralkylcycloalkanen,Perfluoralkanen oder teilweise hydrierten gesättigten oder ungesättigten Fluoralkanen, den Produkten der Formel $C_nF_{2n+1}CH = CH—C_mF_{2m+1}$, (wobei n und m ganze Zahlen sind), heterocyclischen perfluorierten

alkylsubstituierten Verbindungen, tertiären perfluorierten Aminen und perfluorierten Ethern.

4. Verfahren nach einem der Ansprüche 1 bis 3, dadurch gekennzeichnet, daß der Fluorkohlenstoff ausgewählt ist aus :

Perfluordecalin,
Perfluortributylamin,
Perfluortripropylamin,
Perfluormethyldecalin
Perfluoradamantan,
Perfluordimethyladamantan

und Perfluordecan, der Verbindung der Formal $C_8F_{17}CH=CH_2$ oder der Verbindung der Formel $C_8F_{17}CH_2CH_3$.

5. Verfahren nach einem der Ansprüche 1 bis 4, dadurch gekennzeichnet, daß das oberflächenaktive Mittel eine Verbindung der Formel $R_F—CH_2—(OC_2CH_4)_n—OH$ ist,in welcher $R_F$ eine Fluorkohlenstoffkette ist und n mehr als oder gleich 1 ist.

6. Verfahren nach Anspruch 5, dadurch gekennzeichnet, daß man das oberflächenaktive Mittel auswählt aus

$C_7F_{15}(OC_2H_4)_4OH$ ; $C_7F_{15}CH_2(OC_2H_4)_5OH$ ;
$C_7F_{15}CH_2(OC_2H_4)_6OH$ ; $C_6F_{13}CH_2(OC_2H_4)_3OH$ ;
$C_6F_{13}CH_2(OC_2H_4)_4OH$ ; $C_6F_{13}CH_2(OC_2H_4)_5OH$ und
$C_6F_{13}CH_2(OC_2H_4)_6OH$.

7. Verfahren nach einem der Ansprüche 1 bis 6, dadurch gekennzeichnet, daß die um 37 °C stabile Mikroemulsion durch einfaches Mischen von

20 bis 55 Gew.-% Perfluordecalin,
2 bis 15 Gew.-% $C_7F_{15}CH_2(OC_2H_4)_5OH$ und
Wasser

in den durch das ternäre Emulgierungsdiagramm bei 37 °C definierten relativen Verhältnissen hergestellt wird.

8. Verfahren nach einem der Ansprüche 1 bis 6, dadurch gekennzeichnet, daß die um 37 °C stabile Mikroemulsion durch einfaches Mischen von

1 bis 55 Gew.-% $C_8F_{17}CH=CH_2$,
1 bis 18 Gew.-% $C_7F_{15}CH_2(OC_2H_4)_6OH$ und
Wasser

in den durch das ternäre Emulgierungsdiagramm bei 37 °C definierten relativen Verhältnissen hergestellt wird.

9. Verfahren nach einem der Ansprüche 1 bis 6, dadurch gekennzeichnet, daß die um 37 °C stabile Mikroemulsion durch einfaches Mischen von

25 bis 50 Gew.-% $C_8F_{17}CH=CH_2$,
7 bis 15 Gew.-% $C_7F_{15}CH_2(OC_2H_4)_6OH$ und
einer Ringer-Lactatlösung

in den durch das ternäre Emulgierungsdiagramm bei 37 °C definierten relativen Verhältnissen hergestellt wird.

10. Vefahren nach einem der Ansprüche 1 bis 6, dadurch gekennzeichnet, daß die um 37 °C stabile Mikroemulsion durch einfaches Mischens von

1 bis 35 Gew.-% Perfluordecalin,
1 bis 20 Gew.-% $C_6F_{13}CH_2(OC_2H_4)_5OH$ und
Wasser

bei den durch das ternäre Emulgierungsdiagramm bei 37 °C definierten relativen Verhältnissen hergestellt wird.

11. Verfahren nach einem der Ansprüche 1 bis 6, dadurch gekennzeichnet, daß die um 25 °C stabile Mikroemulsion durch einfaches Mischen von

1 bis 30 Gew.-% Perfluordecalin,
1 bis 30 Gew.-% $C_6F_{13}CH_2(OC_2H_4)_5OH$ und
Wasser

in den durch das ternäre Emulgierungsdiagramm bei 25 °C definerten relativen Verhältnissen hergestellt wird.

12. Verfahren, um Organe am Leben zu erhalten, dadurch gekennzeichnet, daß man sie mit einer gemäß einem der Ansprüche 1 bis 11 hergestellten Mikroemulsion, die als Transportmittel für Gase, wie $O_2$, $N_2$, $CO_2$ wirkt, durchströmen läßt.

13. Verfahren zur Herstellung einer Transportlösung von Blutgasen, dadurch gekennzeichnet, daß die Mikroemulsion eine ausreichende Menge Perfluorkohlenstoff für diesen Transport enthält.

FIG.1

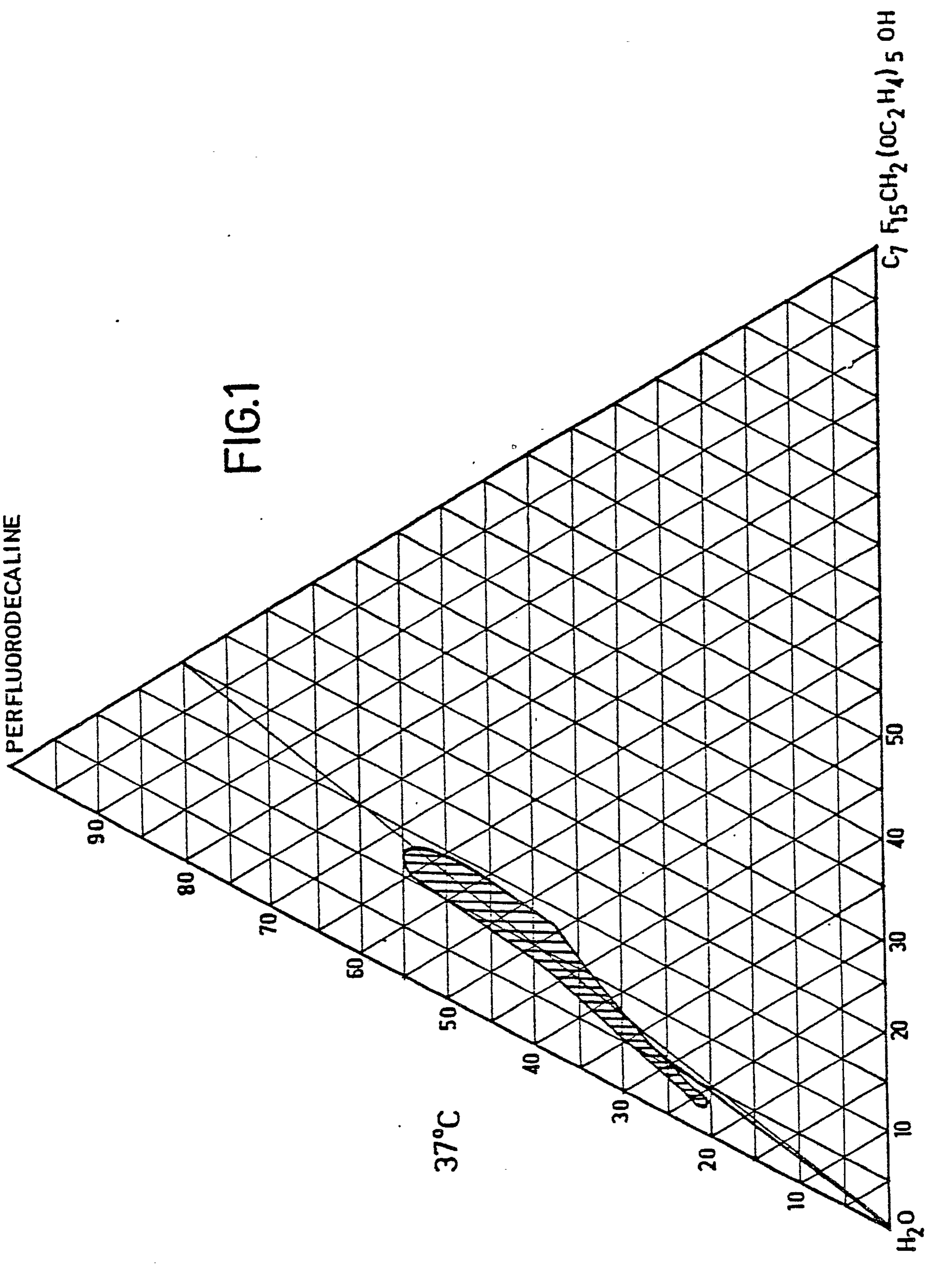

PERFLUORODECALINE
37°C
90
80
70
60
50
40
30
20
10
$H_2O$
10
20
30
40
50
$C_7F_{15}CH_2(OC_2H_4)_5OH$

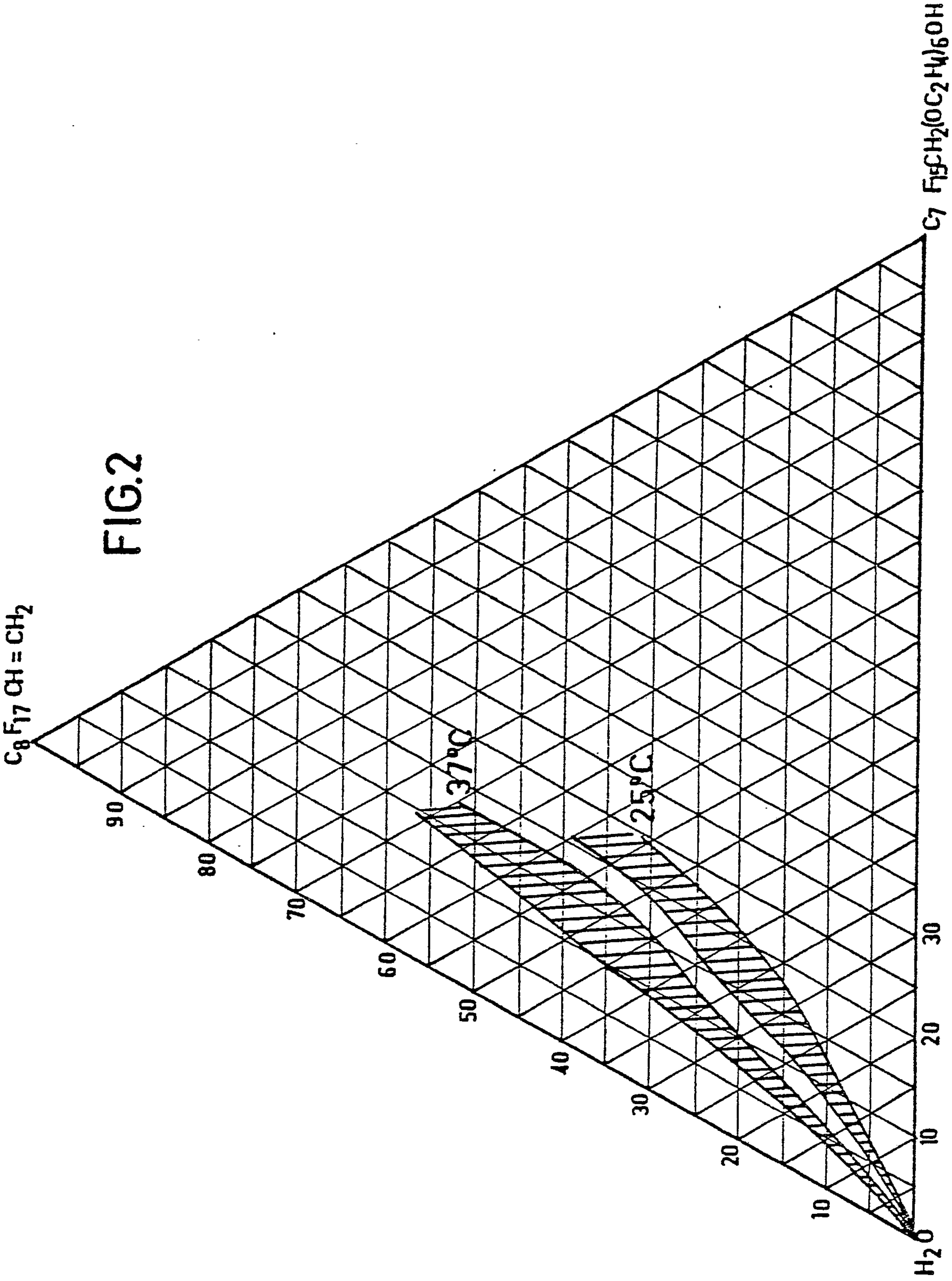
$C_8F_{17}CH=CH_2$
$C_7F_{15}CH_2(OC_2H_4)_6OH$
$H_2O$
37°C
25°C
10
20
30
40
50
60
70
80
90
0
10
20
30

FIG.2

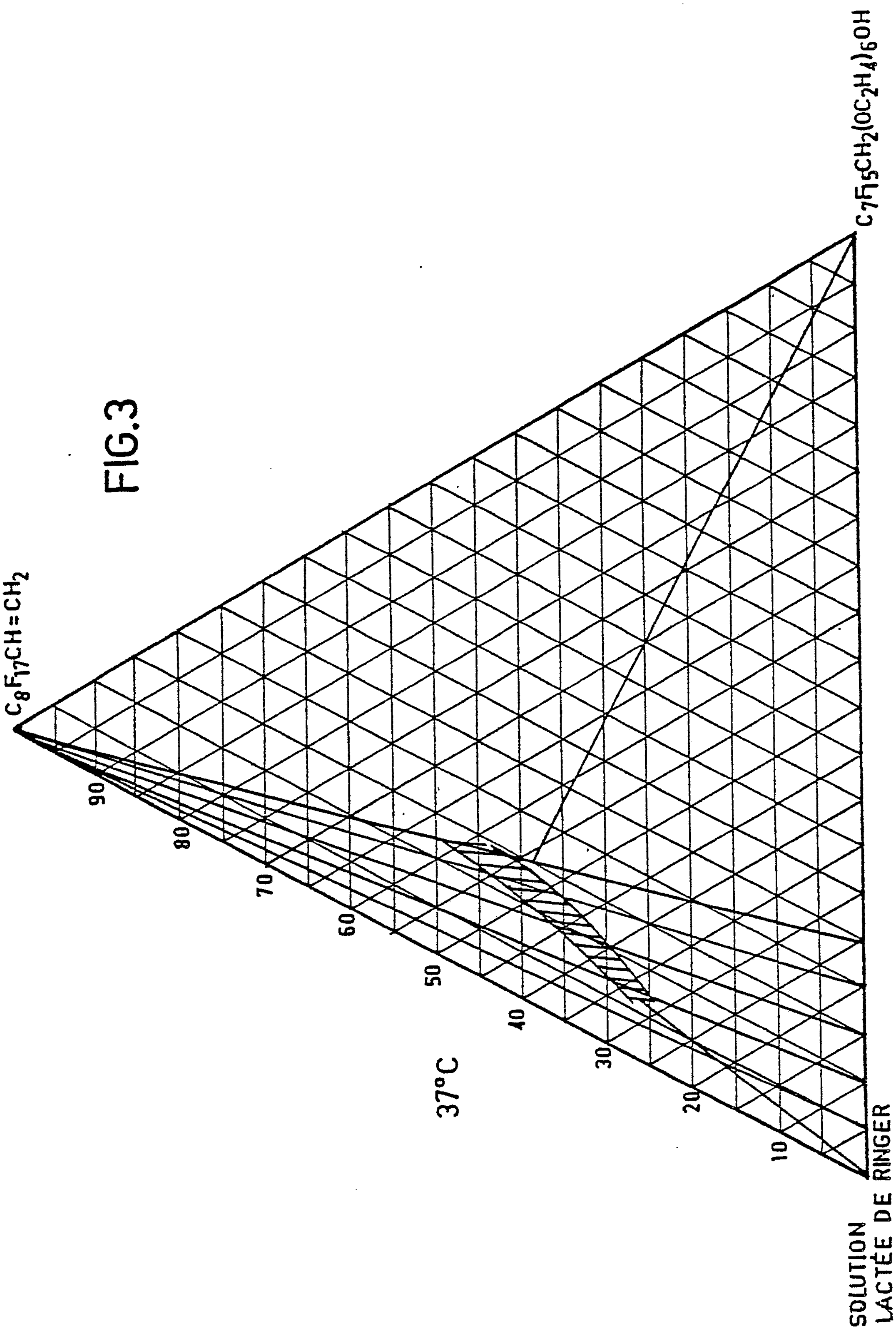

C8F17CH=CH2
C7F15CH2(OC2H4)6OH
SOLUTION LACTÉE DE RINGER
37°C
10
20
30
40
50
60
70
80
90


FIG.3

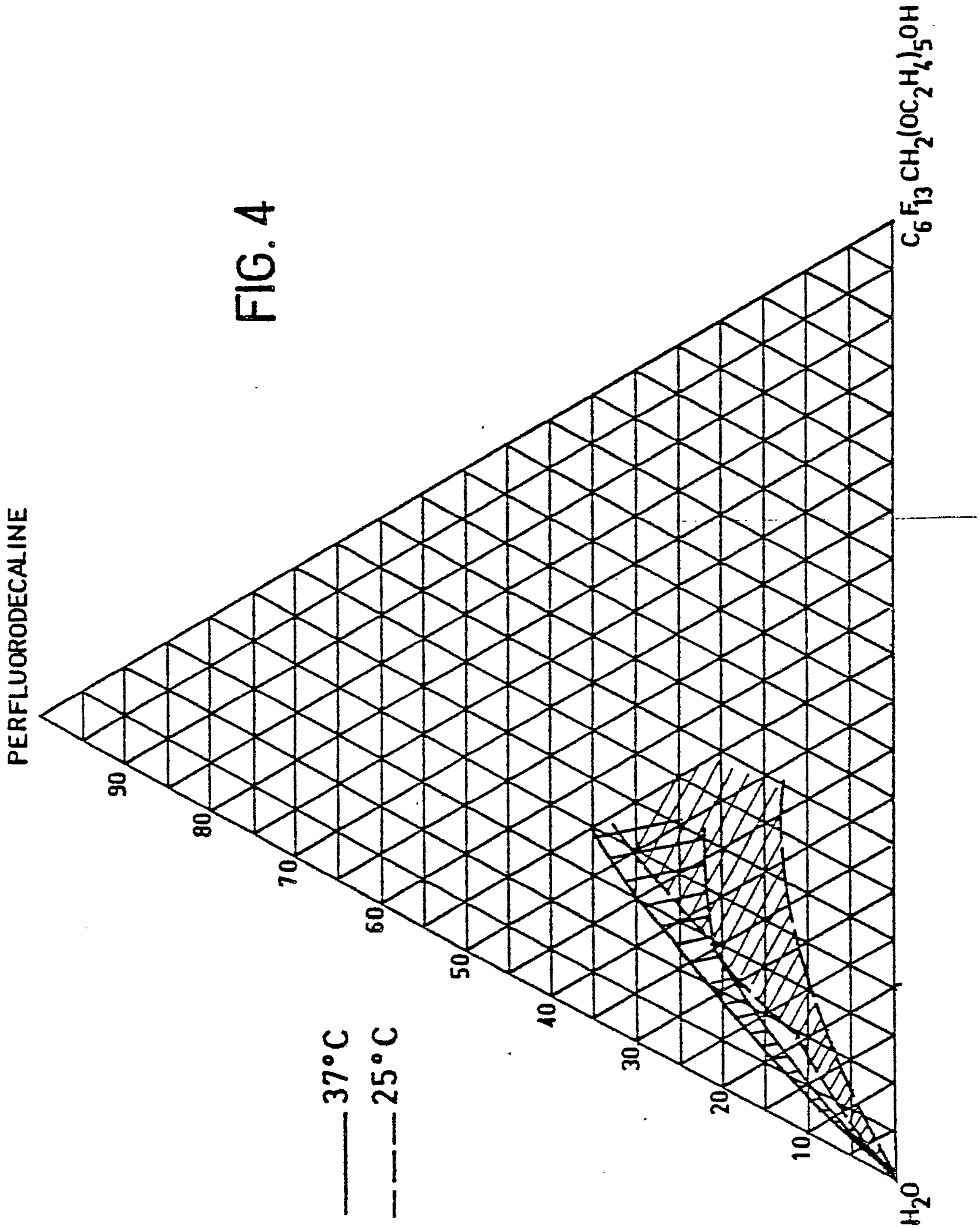

PERFLUORODECALINE
$C_6F_{13}CH_2(OC_2H_4)_5OH$
$H_2O$
10
20
30
40
50
60
70
80
90
37°C
25°C


FIG. 4

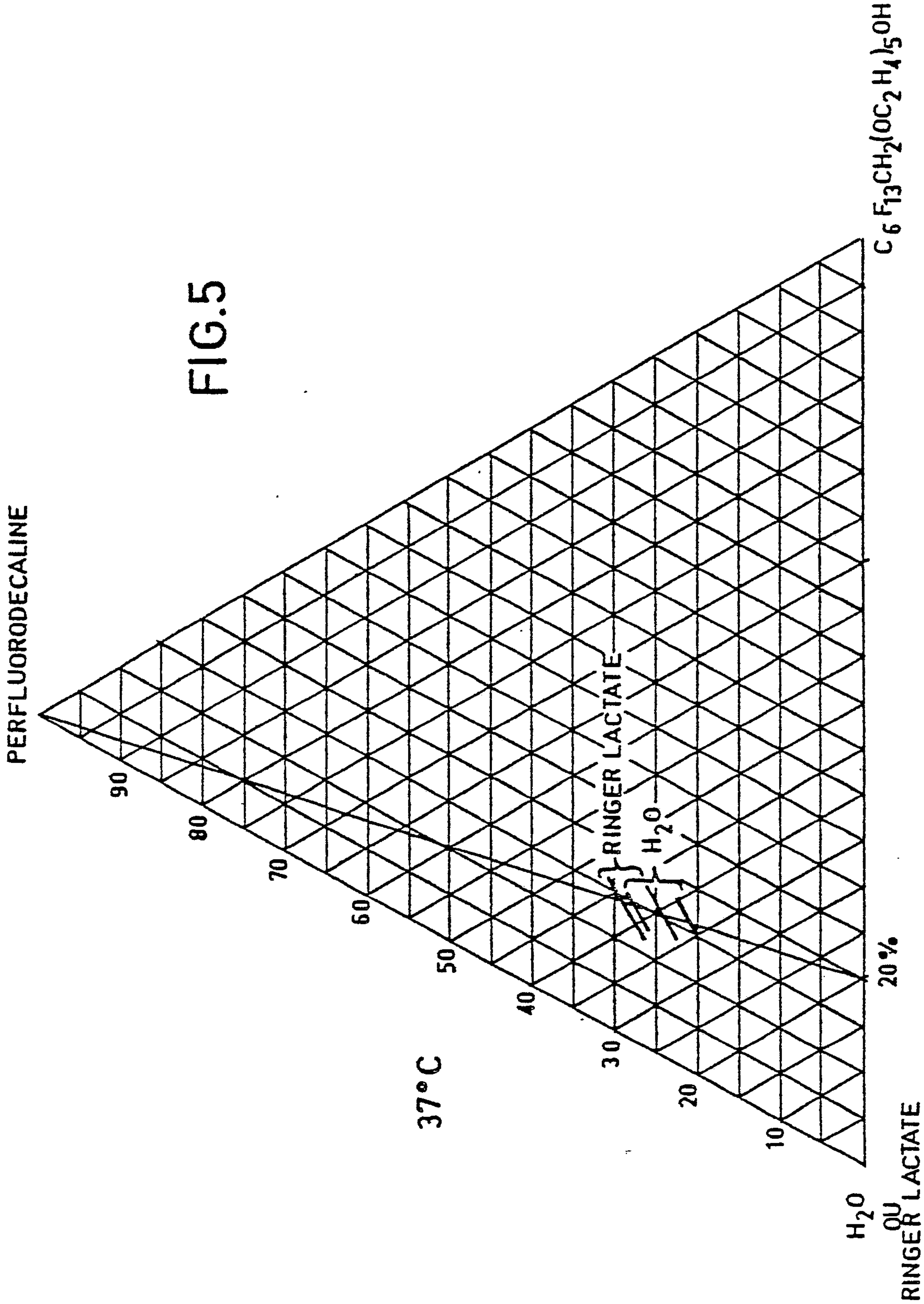
PERFLUORODECALINE
$C_6F_{13}CH_2(OC_2H_4)_5OH$
H$_2$O OU RINGER LACTATE
37°C
90
80
70
60
50
40
30
20
10
20%
RINGER LACTATE
H$_2$O

FIG.5

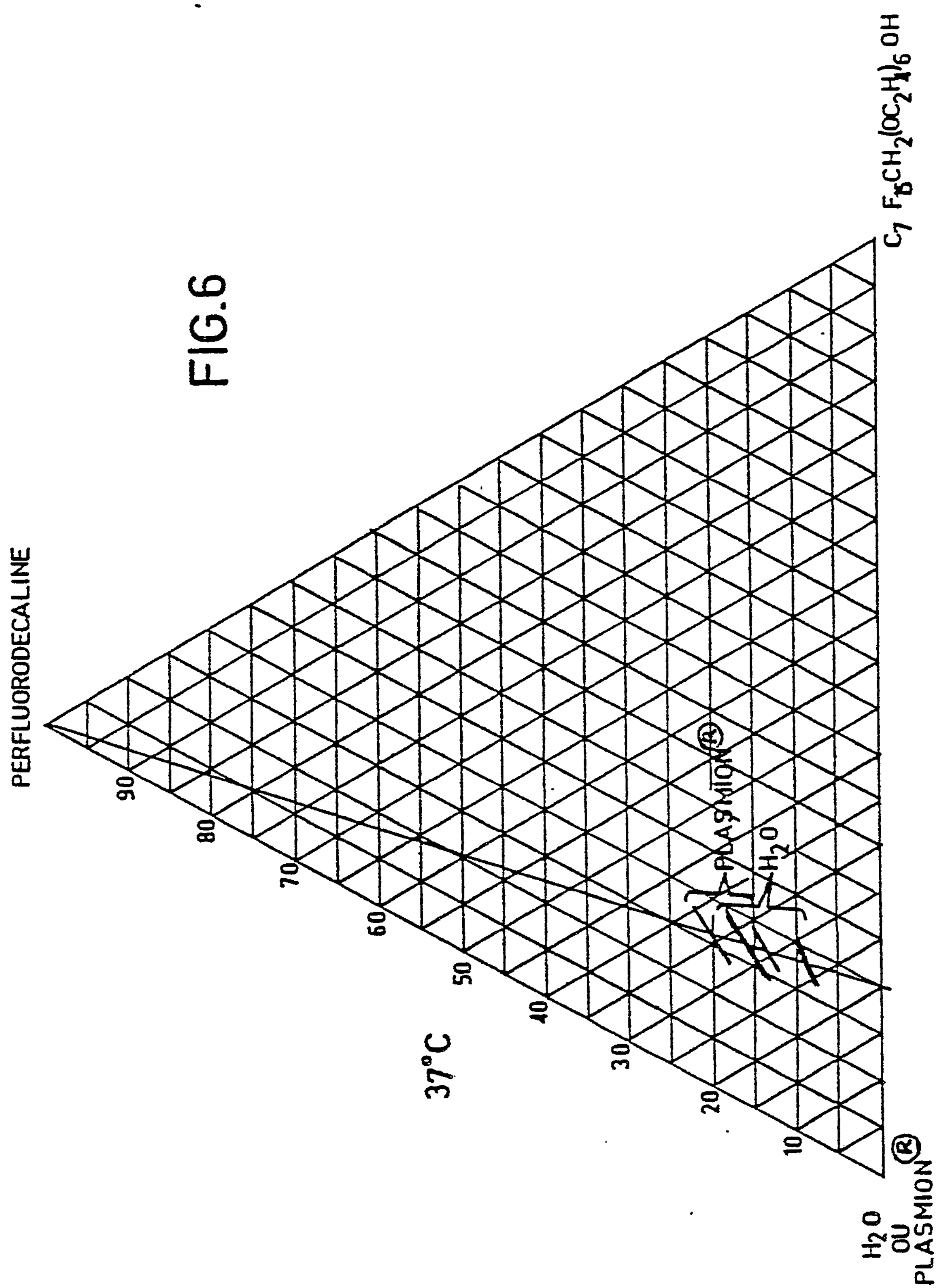
PERFLUORODECALINE
90
80
70
60
50
40
30
20
10
37°C
H2O
OU
PLASMION®
PLASMION®
H2O
C7 F15CH2(OC2H4)6OH

FIG.6